(19) Europäisches Patentamt
European Patent Office
Office européen des brevets

(11) Publication number: **0 187 022 B1**

(12) # EUROPEAN PATENT SPECIFICATION

(45) Date of publication of patent specification: **29.05.91**

(51) Int. Cl.[5]: **C07J 51/00**, G01N 33/78, G01N 33/94

(21) Application number: **85309235.1**

(22) Date of filing: **18.12.85**

The file contains technical information submitted after the application was filed and not included in this specification

(54) **Phospholipid conjugates and their preparation.**

(30) Priority: **28.12.84 US 687135**

(43) Date of publication of application:
**09.07.86 Bulletin 86/28**

(45) Publication of the grant of the patent:
**29.05.91 Bulletin 91/22**

(84) Designated Contracting States:
**BE CH DE FR GB IT LI NL SE**

(56) References cited:
**FR-A- 2 275 483**
**US-A- 3 997 525**
**US-A- 4 235 792**
**US-A- 4 342 826**

**Methods in Enzymology, vol.70 (1980), pp 85-105 .**

(73) Proprietor: **TECHNICON INSTRUMENTS CORPORATION(a Delaware corporation)**
**511 Benedict Avenue**
**Tarrytown, New York 10591-6097(US)**

(72) Inventor: **Hwang, Deng R.**
**52 Tarryhill Road**
**Tarrytown New York 10591(US)**
Inventor: **Scott, Mary E.**
**542 Locust Street**
**Mount Vernon New York 10552(US)**
Inventor: **Hedaya, Eddie**
**5-402 Highpoint Drive**
**Hartsdale New York 10530(US)**

(74) Representative: **Wain, Christopher Paul et al**
**A.A. THORNTON & CO. Northumberland House 303-306 High Holborn**
**London WC1V 7LE(GB)**

EP 0 187 022 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to certain novel immunoreactive phospholipid conjugates useful for homogeneous liposome immunoassays.

Digoxin is a potent cardiac glycoside. Toxic amounts of digoxin exert undesirable and potentially lethal electrophysiological effects (Hoffman et al, The Pharmacological Basis of Therapeutics, Gilman, 6th ed., p 729, N.Y. 1980). Accordingly, various immunoassay methods for cardiac glycosides are now widely used clinically as aids in the determination of appropriate dosage schedules for patients receiving these drugs. Because digoxin is a very small hapten molecule, it is necessary to conjugate it covalently to an antigenic carrier, for example, human serum albumin (HSA), bovine serum albumin (BSA) or keyhole limpet hemocyanin (KLH), in order to elicit digoxin-specific antibodies in experimental animals for use in immunoassay. The preparation of immunoreactive digoxin derivatives is typically carried out by the procedure of Butler et al (Proc. Natl. Acad. Sci., U.S.A., 1967, 57, 71-78; and Methods in Enzymology, Academic Press, 84, 558-577 (1982)) which is based on the work of Erlanger and Beiser, Proc. Natl. Acad. Sci., 52, 68 (1964). The reaction sequence involves periodate cleavage of the terminal sugar ring (digitoxose or rhamnose) followed by reaction with a protein carrier, enzyme or related biological molecule, and finally reductive amination with sodium borohydride. Thus, digoxin-HSA (Butler et al supra), (Smith et al, Biochemistry, 9, 331-337 (1970)), mellitin-ouabain (Freytag et al, J. of Immunological Methods, 70, 133-140 (1984)), and digoxin-dibenzo-18-crown-6 (Keating et al, Anal. Chem., 56, 801-806 (1984)), conjugates have been prepared using the aforementioned reaction sequence.

U.S. patent specification no. 4,115,539 discloses a method of preparing digoxin conjugates using isocyanates, based upon tryrosine methyl ester. U.S. patent specifications nos. 4,297,273 and 4,363,759 disclose ways to prepare chemiluminiscent phthalaldehyde-labelled digoxigenin. U.S. patent specification no. 4,342,826 employs the procedure of Butler et al supra to prepare a non-characterized digoxin-phosphatidylethanolamine conjugate but, when we tried this preparation, a very complex mixture was formed.

FR-A-2275483 describes the preparation of certain derivatives of digoxin by oxidising to a dialdehyde, reacting the dialdehyde with a carboxyalkoxylamine, and then coupling particular amines (eg. histamine and tyrosine) thereto.

We have now discovered certain new digoxin and digoxin-related phospholipid conjugates and other conjugates, which are useful for sensitive liposome immunoassays.

In accordance with one aspect of this invention, there is provided a phospholipid conjugate compound of the formula:

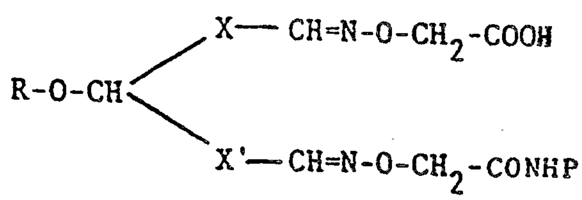

I

or

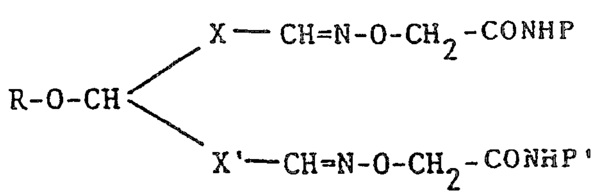

II

wherein R is derived from a steroid containing 1,2-dihydroxy groups which groups are subject to oxidative cleavage; X and X' are the side chain moieties connecting the R moiety to the carbon atoms resulting from oxidative cleavage of the 1,2-dihydroxy groups; and P and P' are phospholipid ethanolamine moieties.

Preferably, R is derived from a cardiac glycoside such as digoxin, digitoxin, gitoxin, ouabain, digitonin and the like. For compounds of Formula I, P is a phospholipid moiety preferably derived from dipalmitoyl

phosphatidyl ethanolamine (DPPE). For compounds of Formula II, P and P' are phospholipid moieties preferably also derived from DPPE. Other suitable phospholipid moieties may be used as desired to introduce modified properties of the liposome. These include the natural phosphatidyl ethanolamines, dimyristyl phosphatidyl ethanolamine, phosphatidyl serine, etc.

In general, the novel phospholipid conjugates of this invention are derived from compounds having functional groups which can be modified and/or converted to form oxime derivatives.

The invention also includes a liposomal immunoassay, such as that disclosed in U.S. patent specifications nos. 4,342,826 or 4,235,792, to which reference should be made for further details, wherein there is used a phospholipid conjugate of the invention.

The invention further includes a method of preparing a phospholipid conjugate of the invention, which method comprises the steps of oxidatively cleaving the 1,2-dihydroxy groups of the steroid, reacting the resulting 1,2-dialdehyde groups with carboxy methoxylamine to form the corresponding dioxime, and preparing the phospholipid conjugate by reacting phospholipid with said dioxime.

An example of this method is shown, using digoxin by way of illustration, in Scheme A provided hereinafter.

Scheme A

Synthesis Of Digoxin-DPPE Conjugates

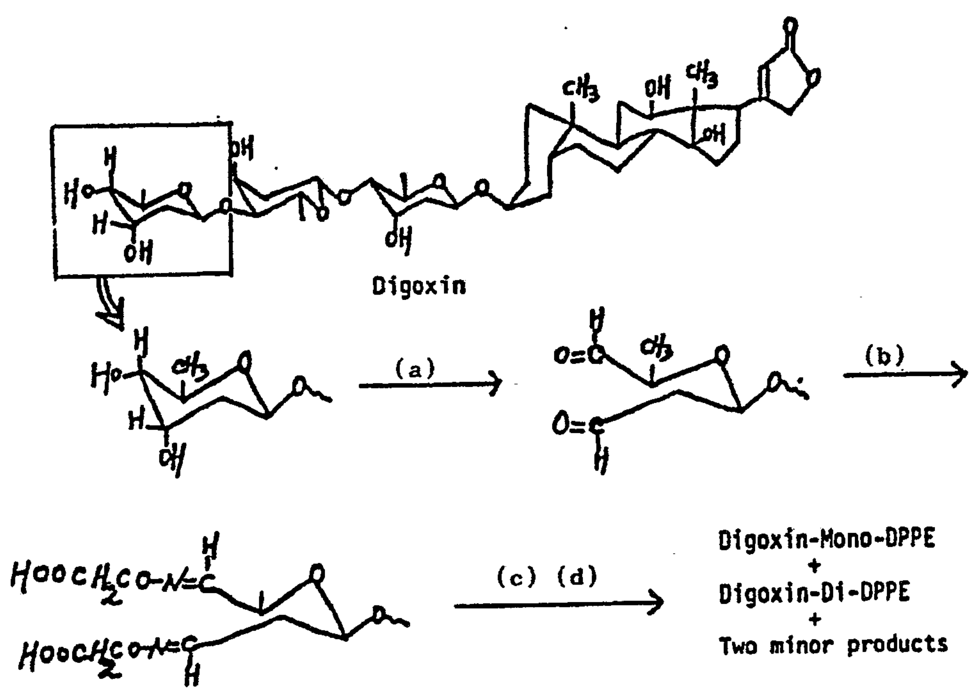


(a) sodium periodate
(b) $(NH_2OCH_2COOH)_2.HCl/NaOAc$ + EtOH
(c) N-hydroxysuccinimide (NHS) + dicyclohexylcarbodiimide (DCC)→ active ester
(d) dipalmitoyl phosphatidyl ethanolamine/$CHCl_3/Et_3N$

In step (c):

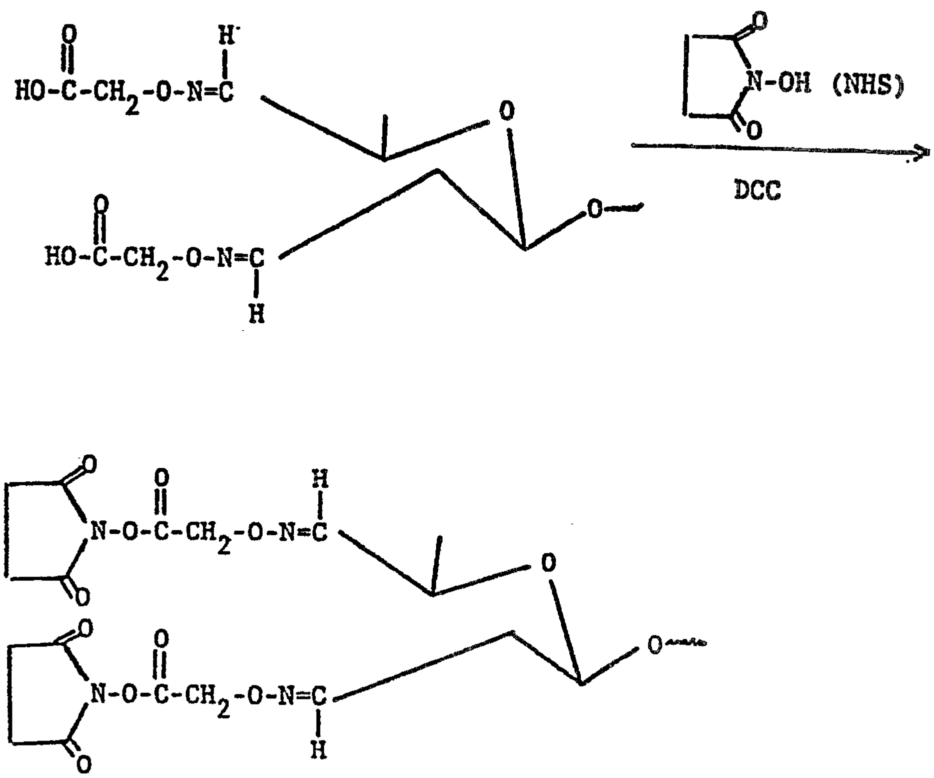


N-hydroxysuccinimide ester of digoxin di-(o-carboxymethyl) oxime ("active ester"). In step (d), di-DPPE and mono-DPPE conjugates are formed as follows:

"active ester" —— $DPPE^*/CHCl_3/Et_3N$ ——→ -mono-DPPE + -di-DPPE

wherein the mono-compound has the formula

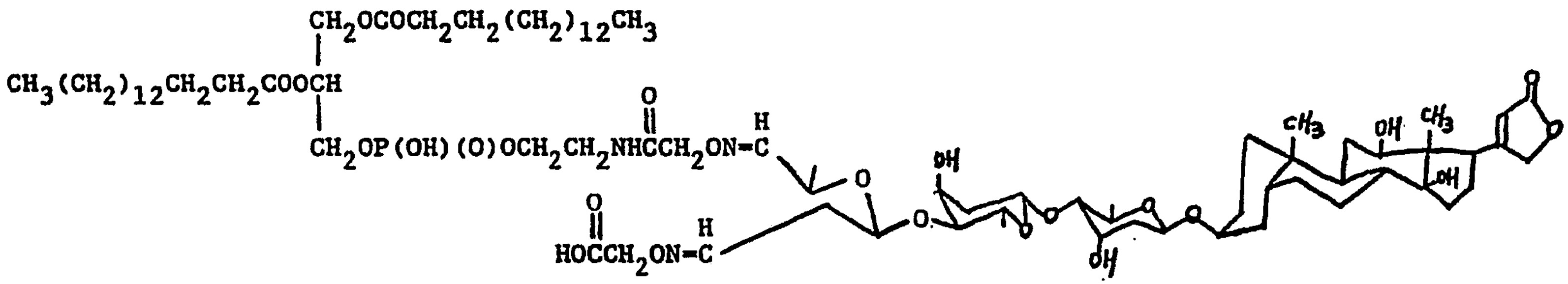

---

*DPPE has the formula:

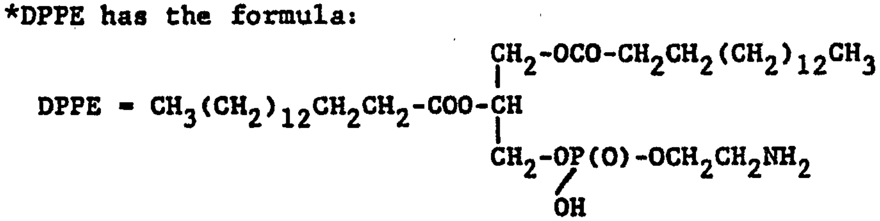

and the di-compound has the formula

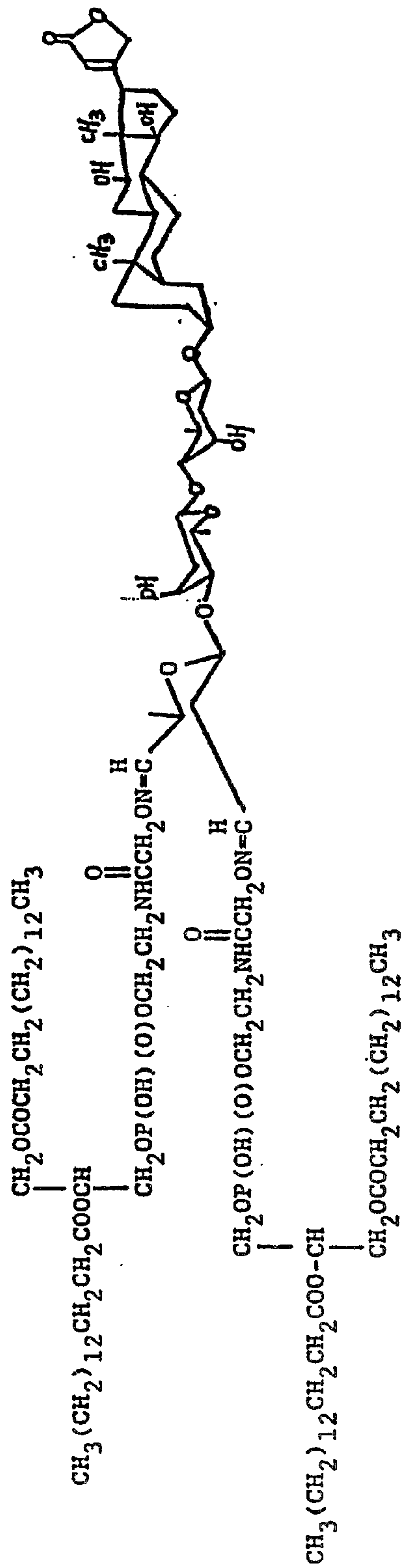

In the above reaction sequence, the terminal digitoxose in digoxin is cleaved to give dialdehyde in quantitative yield by using, for example, sodium periodate under a nitrogen atmosphere. Thin layer chromatography (silica gel, Merck) using a chloroform/methanol (10/1 by volume) solvent system shows one homogeneous spot at $R_f$ 0.16 (detected by spraying with methanol/concentrated sulfuric acid (9/1 by volume) and warming briefly to develop a dark brown color).

The condensation reaction of digoxin dialdehyde and carboxy methoxyamine hemihydrochloride proceeds rapidly in sodium acetate/ethanol under a nitrogen atmosphere. Quantitative yields of di-(0-carboxymethyl) oxime (TLC: $R_f$ 0.04-0.12 solvent system chloroform/methanol, 6/1 by volume) were obtained. The digoxin dioxime derivative was used immediately in the next reaction step. The carboxy functionalities of the dioxime are reacted with N-hydroxysuccinimide in the presence of dicyclohexylcarbodiimide to give an active ester. The dioxime active ester is then condensed with dipalmitoyl phosphatidyl ethanolamine (DPPE) with gentle heating for seventy two hours. The reaction is monitored closely by TLC. Thin layer chromatography with the solvent system chloroform/methanol/water (75/25/3 by volume) showed two major components at $R_f$ 0.20 and 0.13 along with N-hydroxysuccinimide at $R_f$ 0.30. The phospholipid moiety of the conjugates was detected by molybdate blue spray. The excess N-hydroxysuccinimide was removed from the reaction mixture by preparative liquid chromatography (Kieselgel, 200g, glass column (2.5 x 50 cm), solvent system chloroform/methanol/water (2/8/1 by volume)). Pure digoxin-di-DPPE conjugate (0.1348 g, 20%; $R_f$ 0.30) and digoxin-mono DPPE conjugate (0.1424 g, 26.5% $R_f$ 0.15) were isolated from the crude reaction mixture by preparative TLC in chloroform/methanol/water (2/8/1 by volume). Two other minor products were also isolated. The structures of the minor products were not identified.

The proof of the structure of the two major conjugates was made using the IR, UV and high resolution proton NMR spectra, and fast atom bombardment (FAB) mass spectra.

The advantages of the above described synthetic procedure reside in its ability and facility efficiently to provide a relatively stable, storable yet activatable, oxime intermediate. This intermediate overcomes the disadvantages inherent in the Butler et al procedure discussed earlier. These include the propensity for the dialdehyde intermediate to undergo deleterious side-reactions, particularly in the presence of amine derivatives of lesser reactivities such as phospholipids. An additional advantage is that the procedure of the present invention provides products which can be readily isolated, characterised and purified in contrast to Butler which, to our knowledge, yields a sufficiently complex mixture to thwart the desired product characterization.

The synthetic method described herein is also applicable to the preparation of analogous phospholipid conjugates involving linkage through a sugar ring such as digitoxin, gitoxin, ouabain, digitonin and related cardiac glycosides, or those involving steroids which can be modified, to form oxime derivatives remote from key functionalities important for immunorecognition by specific antibodies.

In order that the invention may be more fully understood, the following Examples are given by way of illustration only.

## EXAMPLE I

### Synthesis of Digoxin DPPE Conjugates

#### (A) Digoxin Dialdehyde

Digoxin (0.4985 g, 0.64 mmole) is dissolved in 10 ml of chloroform/methanol (3/1.5) and placed into a 100 ml two-necked flask. Sodium periodate (0.3102 g, 1.4 mmoles) is dissolved in 4 ml distilled water and placed into a pressure equalized addition funnel. The periodate solution is slowly added to the flask while stirring and under nitrogen. A white precipitate is immediately formed and the reaction is complete within 15 minutes after addition of the periodate. Reaction progress is monitored by TLC (E.M. Merck, pre-coated TLC sheets, silica gel 60 F254 0.2 mm thickness) in chloroform/methanol 10/1 by volume ($R_f$ 0.16 = dialdehyde, one homogeneous spot; $R_f$ 0.07 = digoxin). Both spots become dark brownish when the TLC plate is sprayed with methanol/concentrated sulfuric acid (9/1 by volume) and placed in 100$^\circ$C oven). The reaction mixture is evaporated on a rotary evaporator and brought up in 30 ml of chloroform and 3 ml of water. The cloudy solution is extracted and the aqueous layer washed three times with 10 ml chloroform. The organic phases are combined (60 ml) and dried over magnesium sulfate. The organic solvents are evaporated to dryness. A light yellow brownish oily material is left. This material is used immediately in the next stage.

#### (B) Digoxin Di(0-carboxymethyl) Oxime

Carboxy methoxylamine hemihydrochloride (0.3119 g, 1.4 mmoles) and sodium acetate (0.2260 g, 1.6 mmoles) are dissolved in 3 ml water and placed into a 50 ml 2-necked flask. The digoxin dialdehyde, dissolved in 1.3 ml ethanol, is placed into a pressure equalized funnel and slowly added to the flask with stirring and under nitrogen. The reaction is complete within ten minutes (TLC: chloroform/methanol 6/1 by volume, $R_f$ 0.09-0.13). The reaction mixture is evaporated to dryness and dissolved in 20 ml ethyl acetate and 3 ml water. The organic layer is separated and the aqueous layer is washed three times with 5.0 ml ethyl acetate. The organic layers are combined and dried over anhydrous magnesium sulfate. The solution is filtered and evaporated to dryness. The residue is dried for 30 minutes under high vacuum (0.1 mm Hg) and used immediately for the next step.

(C) Preparation of N-Hydroxysuccinimide Ester of Digoxin Di(0-Carboxymethyl) Oxime

Dicyclohexylcarbodiimide (DCC) (0.2805 g, 1.3 mmoles) is dissolved in 6 ml dry DMF (dimethyl sulfoxide) and placed into a 50 ml 2-necked flask. The solution is cooled in an ice-water bath (4° C). Digoxin di(0-carboxymethyl) oxime, dissolved in 80 ml DMF, is slowly added to the flask with stirring and under nitrogen. Immediately afterwards, N-hydroxysuccinimide solution (0.1500 g, 1.3 mmoles, in 6 ml DMF) is likewise added. Reaction progress is monitored by TLC (chloroform/methanol/water 75/25/3 by volume, $R_f$ 1.0 (DCC), 0.75 (dioxime NHS active ester), 0.34 (NHS), 0.1 (dioxime)). The reaction is continued at 4° C under nitrogen for 18 hours.

The desired product possesses the following charactistics in TLC: (1) homogeneous UV detectable spot (short wavelength), (2) the homogeneous spot turns brownish when spraying the TLC plate with methanol/concentrated sulfuric acid (9/1 by volume) and warming the plate briefly in 100° C oven.

The reaction mixture is filtered to remove dicyclohexyl urea and the crude reaction mixture is used for the next step.

(D) Preparation of Digoxin-DPPE Conjugates

The crude dioxime active ester (17 ml reaction mixture) is placed into 100 ml 2-necked flask. A suspension of DPPE (0.4431 g, 0.64 mmoles, dispersed in 30 ml dry chloroform and 0.7 ml of triethylamine) is placed in an addition funnel and slowly added to the flask while stirring under nitrogen and being protected from light. The mixture is heated gently (40-50° C) for 72 hours. The reaction is monitored by TLC (solvent system chloroform/ methanol/water 75/25/3 by volume; $R_f$ 0.75 (active ester), 0.52 (unknown 1), 0.45 (unknown 2), 0.30 (NHS), 0.21 DPPE, 0.20 (disubstituted conjugate), 0.13 (mono-substituted conjugate). The reaction mixture is complex and the above are the major identified products. The phospholipid moiety of the conjugates was detected by molybdate blue spray. The reaction mixture was evaporated and brought up in 10 ml chloroform/methanol/water (2/8/1). The N-hydroxysuccinimide was removed from the mixture by LPLC ((Kiesegel 200 g, glass column (2.5 cm x 50 cm), solvent system chloroform/methanol/water (2/8/1 by volume)). Pure mono and disubstituted conjugates can be obtained by preparative TLC in chloroform/methanol/ water (2/8/1 by volume, $R_f$ 0.30 (disubstituted); 0.15 (monosubstituted)). Pure digoxin-di-DPPE conjugate (0.1349 g 20%) and digoxin-mono-DPPE conjugate (0.1424 g 26.5%) were obtained. Two other minor products were also isolated. The structures of the minor products were not identified.

E) NMR, UV, IR and FAB Data

The NMR, UV and IR and fast atom bombardment (FAB) mass spectra data are summarized in Chart 1 and 2 provided hereinbelow:

CHART 1

Digoxin-Mono-DPPE

NMR (300 MHz, $CDCl_3$)

δ 0.8 (singlet, 3H, 18$CH_3$), 0.96 (singlet, 3H, 19 $CH_3$),
0.90 (triplet,6H, terminal methyl group in phospholipid)
2.23-1.05 [ (complex multiplet, 83H, 2 $(CH_2)_{12}$, 3 $CH_3$(digitoxose ring), 3 $CH_2$(digitoxose ring), 8 $CH_2$(digoxigenin ring), 2 $\underline{CH_2}(CH_2)_{12}$ ]
2.32 (two overlapping triplets, 4H, 2 $CH_2CO$)
4.72-3.05 [ (complex multiplet, 27H, $CH_2OCOR$, 9 CH (digitoxose ring proton), 6 CH (digoxigenin ring proton), glycero $CH_2$-O-P protons, ethanolamine $CH_2$-O-P, -$OCH_2$-CO- and -$CH_2NH_2$)]
4.95 (multiplet, 5H, $CH_2$ in lactone and 3 protons at $C_1$, $C_{1'}$, and $C_{1''}$ in digitoxose)
5.25 (multiplet, 1H, -CH-OCOR)
5.95 (singlet, 1H, lactone,-C=CH)

UV (Cary 219, $CHCl_3$) max. 241 nm (ε 1744)
IR (KBr, Perkin Elmer 1430 ratio reading, $cm^{-1}$) : 3435 (broad OH), 2923, 2852, 1743 (ester), 1668, 1622.

CHART 2

Digoxin-Di-DPPE

NMR (300MHz,$CDCl_3$)

δ 0.82 (singlet, 3H, 18$CH_3$), 0.94 (singlet, 3H, 19 $CH_3$),
0.90 (triplet, 12H, terminal methyl group in phospholipid)
2.32 (multiplet, 8H, 4 $CH_2$-CO
2.2-1.05 (complex multiplet)
4.75-2.8 (complex multiplet)
4.9 (multiplet, 5H, $CH_2$ in lactone and 3 protons at $C_1$, $C_{1'}$,and $C_{1''}$ in digitoxose )
5.25 ( multiplet, 2H, 2 CH-OCOR)
5.95 (singlet,1H, Lactone -C=CH)

UV (Cary 219, $CHCl_3$) max. 241 nm (ε 2071)
IR (KBr, Perkin Elmer 1430 ratio reading, $cm^{-1}$) : 3427 (broad OH) 2923, 2853,1781,1743,1668.

As a further confirmation of the structure, positive ion fast atom bombardment (FAB) mass spectra (m/Z) from purified conjugates in a thioglycerol matrix were obtained using the MS-50 high resolution mass spectrometer. The most intense peak appeared in the molecular ion region, representing m/Z of $(M + metal)^+$ and the isotopically enriched species. The molecular ion for digoxin-mono-DPPE is 1621 $(M + Na)^+$ and the molecular ion for digoxin-di-DPPE is 2311 $(M + K)^+$.

(F) Liposomal Immunoassay

Both conjugates may be used in the liposomal immunoassay methods described in U.S. patent specifications nos. 4,342,826 (e.g. Example X) and 4,235,792 with acceptable results.

EXAMPLE II

The procedure of Example I is repeated using, in place of the digoxin, approximately stoichiometric equivalent amounts of the following compounds:
digitoxin
gitoxin
ouabain
digitonin
Comparable results were obtained.

EXAMPLE III

The procedure of Example I is repeated using, in place of dipalmitoyl phosphatidyl ethanolamine, an approximately stoichiometric equivalent amount of dimyristyl phosphatidyl ethanolamine. Corresponding products were obtained.

It will be seen that this invention provides a rapid and efficient approach to digoxin and digoxin-related phospholipid conjugates which are useful for liposome immunoassay. A synthetic procedure is provided which involves the linking of a terminal sugar group, e.g. digitoxose of digoxin, to a phospholipid through a carboxymethyl oxime functionality. Such procedure provides much improved yields of readily purified products compared to known procedures. Moreover, it is applicable to related phospholipid conjugates where linkage through a sugar ring is highly preferred, i.e. digitoxin, gitoxin, ouabain, digitonin and other related cardiac glycosides. In addition to these cardiac glycosides, other glycosides including those from the saponin class, but not limited to that class, are within the purview of this invention.

**Claims**

**1.** A phospholipid conjugate compound of the formula:

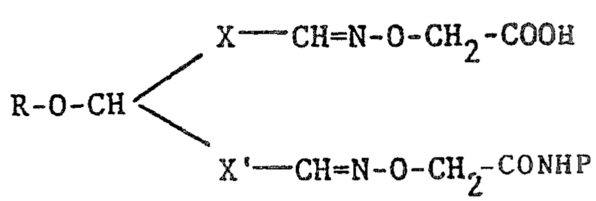

I

or

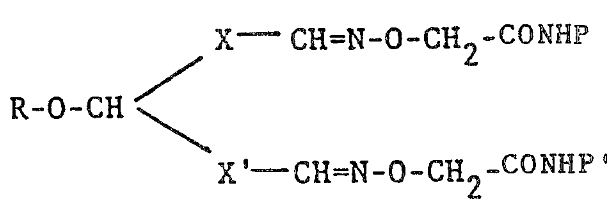

II

wherein R-O- designates a steroid which contains a 1,2-dihydroxy group which group is subject to oxidative cleavage; X and X' are the side chain moieties connecting the R moiety to the carbon atoms resulting from oxidative cleavage of the 1,2-dihydroxy groups; and P and P' are phospholipid ethanolamine moieties.

2. A compound according to claim 1, wherein R is derived from digoxin, digitoxin or gitoxin, X is

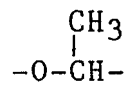

and X' is $-CH_2-$.

3. A compound according to claim 2, wherein P in Formula I, or P and P' in Formula II, are derived from dipalmitoyl phosphatidyl ethanolamine (DPPE).

4. A compound according to claim 2, wherein P in Formula I, or P and P' in Formula II are derived from dimyristyl phosphatidyl ethanolamine.

5. A compound according to claim 1, wherein R is derived from ouabain, X is

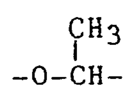

or

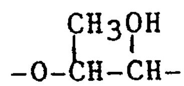

and X' is

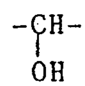

or nothing, and mixtures of two or more such compounds.

6. A compound according to claim 5, wherein P in Formula I, or P and P' in Formula II are derived from dipalmitoyl phosphatidyl ethanolamine (DPPE).

7. A method of preparing a compound according to claim 1, having Formula I or Formula II, which comprises the steps of oxidatively cleaving the 1,2-dihydroxy groups of the steroid, reacting the resulting 1,2-dialdehyde groups with carboxy methoxylamine to form the corresponding dioxime, and preparing the phospholipid conjugate by reacting phospholipid with said dioxime.

8. A method according to claim 7, wherein a mixture containing compounds of Formulae I and II is prepared.

9. A method according to claim 7, wherein there is prepared a phospholipid conjugate as claimed in claim 1 wherein A is derived from digoxin, and P is derived from dipalmitoyl phosphatidyl ethanolamine (DPPE).

10. A method according to claim 7, wherein there is prepared a phospholipid conjugate wherein R is derived from digitoxin, gitoxin, ouabain or digitonin, and P, or P and P' , are derived from dipalmitoyl phosphatidyl ethanolamine (DPPE).

11. A liposomal immunoassay for the determination of appropriate dosage schedules of cardiac glycosides and steroids for human administration, which employs a phospholipid conjugate as claimed in claim 1.

## Revendications

1. Composé de conjugués de phospholipides de formule :

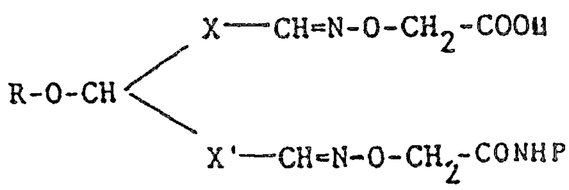

I

ou

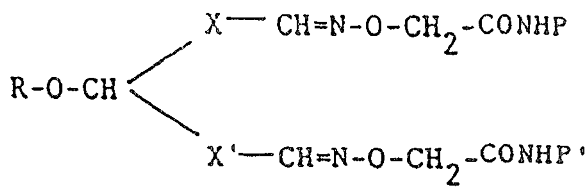

II

où R-O- représente un stéroïde qui contient un groupe 1,2-dihydroxy, lequel groupe est soumis à un clivage par oxydation ; X et X' représentent les fragments de chaîne latérale reliant le fragment R aux atomes de carbone résultant du clivage par oxydation des groupes 1,2-dihydroxy ; P et P' représentent des fragments d'éthanolamine de phospholipides.

2. Composé selon la revendication 1, caractérisé en ce que R est dérivé de la digoxine, de la digitoxine ou de la gitoxine, X représente

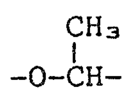

et X' représente $-CH_2-$.

3. Composé selon la revendication 2, caractérisé en ce que P, dans la formule I, ou P et P', dans la formule II, sont dérivés de la dipalmitoyl-phosphatidyl-éthanolamine (DPPE).

4. Composé selon la revendication 2, caractérisé en ce que P, dans la formule I, ou P et P', dans la formule II, sont dérivés de la dimyristyl-phosphatidyl-éthanolamine.

5. Composé selon la revendication 1, caractérisé en en ce que R est dérivé de l'ouabaïne, X représente

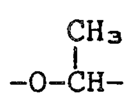

ou

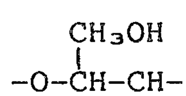

et X' représente

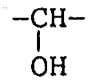

ou rien, et des mélanges de deux ou plusieurs composés de ce type.

6. Composé selon la revendication 5, caractérisé en ce que P, dans la formule I, ou P et P', dans la formule II, sont dérivés de la dipalmitoyl-phosphatidyl-éthanolamine (DPPE).

7. Procédé de préparation d un composé selon la revendication 1, de formule I ou de formule II, caractérisé en ce qu'il comprend les phases de clivage par oxydation des groupes 1,2-dihydroxy du stéroïde, la réaction des groupes 1,2-dialdéhyde résultants avec une carboxyméthoxylamine, pour former la dioxime correspondante, et la préparation du conjugué de phospholipides en faisant réagir le phospholipide avec ladite dioxime.

8. Procédé selon la revendication 7, caractérisé en ce qu'on prépare un mélange contenant des composés de formules I et II.

9. Procédé selon la revendication 7, caractérisé en ce qu'on prépare un conjugué de phospholipides selon la revendication 1, dans lequel R est dérivé de la digoxine, et P est dérivé de la dipalmitoyl-phosphatidyl-éthanolamine (DPPE).

10. Procédé selon la revendication 7, caractérisé en ce qu'on prépare un conjugué de phospholipides dans lequel R est dérivé de la digitoxine, de la gitoxine, de l'ouabaïne ou de la digitonine, et P, ou P et P', sont dérivés de la dipalmitoyl-phosphatidyl-éthanolamine (DPPE).

11. Immunoessai liposomal pour la détermination de modèles de posologie appropriée de glucosides cardiotoniques et de stéroïdes pour une administration chez l'homme, caractérisé en ce qu'il met en oeuvre un conjugué de phospholipides selon la revendication 1.

**Ansprüche**

1. Phospholipidkonjugat der Formel

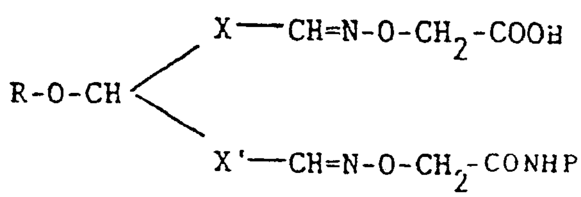

I

oder

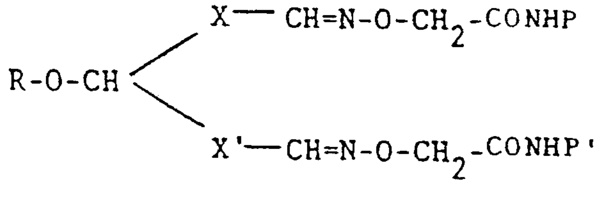

II

worin R-O- ein Steroid mit einer 1,2-Dihydroxygruppierung bedeutet, die einer oxydativen Aufspaltung

unterliegt; X und X' Seitenkettenreste bedeuten, die den Rest R mit den Kohlenstoffatomen verbinden, die sich aus der Spaltung der 1,2-Dihydroxygruppierung ergeben; und P und P' Phospholipidethanolamin-Reste bedeuten.

2. Verbindung gemäß Anspruch 1, worin R von Digoxin, Digitoxin oder Gitoxin abgeleitet ist, X eine Gruppe der Formel

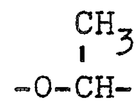

und X' eine $-CH_2$-Gruppe bedeuten.

3. Verbindung gemäß Anspruch 2, worin P in Formel I oder P und P' in Formel II von Dipalmitoylphosphatidylethanolamin (DPPE) abgeleitet sind.

4. Verbindung gemäß Anspruch 2, worin P in Formel I oder P und P' in Formel II von Dimyristylphosphatidylethanolamin abgeleitet sind.

5. Verbindung gemäß Anspruch 1, worin R von Ouabain abgeleitet ist, X den Rest

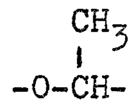 oder 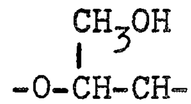

bedeutet und X' den Rest

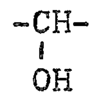

oder nichts bedeutet, sowie Gemische zweier oder mehrerer derartiger Verbindungen.

6. Verbindung gemäß Anspruch 5, worin P in Formel I oder P und P' in Formel II von Dipalmitoylphosphatidylethanolamin (DPPE) abgeleitet sind.

7. Verfahren zur Herstellung einer Verbindung gemäß Anspruch 1 mit der Formel I oder der Formel II, das darin besteht, daß man die 1,2-Dihydroxygruppierung des Steroids oxydativ spaltet, die erhaltenen 1,2-Dialdehydgruppen mit Carboxymethoxylamin zu dem entsprechenden Dioxim umsetzt und das Phospholipidkonjugat durch Umsetzung eines Phospholipids mit dem Dioxim herstellt.

8. Verfahren gemäß Anspruch 7, wobei ein Gemisch hergestellt wird, das die Verbindungen der Formeln I und II enthält.

9. Verfahren gemäß Anspruch 7, wobei ein Phospholipidkonjugat hergestellt wird, wie in Anspruch 1 beansprucht, bei dem R von Digoxin und P von Dipalmitoylphosphatidylethanolamin (DPPE) abgeleitet sind.

10. Verfahren gemäß Anspruch 7, wobei ein Phospholipidkonjugat hergestellt wird, bei dem R von Digitoxin, Gitoxin, Ouabain oder Digitonin und P oder P und P' von Dipalmitoylphosphatidylethanolamin (DPPE) abgeleitet sind.

11. Liposomaler Immunoassay zur Bestimmung geeigneter Dosierungspläne für Herzglycoside sowie

Steroide für die menschliche Behandlung, der sich eines Phospholipidkonjugats gemäß Anspruch 1 bedient.